# EUROPEAN PATENT APPLICATION

(11) **EP 3 750 994 A1**
(43) Date of publication of application: **16.12.2020**
(21) Application number: 20732091.2
(22) Date of filing: 31.03.2020
(51) Int. Cl.: C12N 15/10

(54) **METHOD FOR PURIFYING AND CONCENTRATING DNA IN FORENSIC SAMPLES USING SELECTIVE FILTRATION COLUMN**

(30) Priority: 19.04.2019 CN 201910319898
(71) Applicant: Anhui Senpeng Biotechnology Co., Ltd., Hefei, Anhui 230000 (CN)
(72) Inventor: ZHANG, Dan, Hefei, Anhui 230000 (CN); XIONG, Huai, Hefei, Anhui 230000 (CN); LIANG, Longjie, Hefei, Anhui 230000 (CN)
(74) Representative: Gong, Jinping
(86) International application number: PCT/CN2020/082364
(87) International publication number: WO 2020/211637

(57) **Abstract**

The invention discloses a method for purifying and concentrating DNA in a forensic sample by using a selective filtration column, including the steps of: adding cytosol after digestion and lysis into an inner tube of a selective filtration column, and centrifuging the selective filtration column in a centrifuge; adding 5-100 microliters of water into the selective filtration column, shaking for 30 seconds, and transferring an aqueous solution in the selective filtration column into a clean centrifuge tube, in which case a solution in the centrifuge tube is a purified and concentrated. According to the method, a lysis solution is used for lysing and digesting residual human cells in the sample, after this, the DNA is completely free in the solution, solid particles in the sample are centrifugally removed through the centrifuge tube, and then the solid particles pass through an ultrafiltration membrane at the bottom of the selective filtration column, under the condition of high-speed centrifugation, the ultrafiltration membrane is only permeable to small molecular substances and impermeable to bio-macromolecular substances, such as DNA, so that the purification and concentration of the macromolecular substance, i.e., DNA, can be realized.

## Description

### Technical Field

The invention relates to the field of forensic material evidence identification, and in particular to a method for purifying and concentrating DNA in a forensic sample by using a selective filtration column.

### Background Art

Forensic DNA typing is one of the important technical means in forensic material evidence identification. Common forensic DNA typing techniques include restriction fragment length polymorphisms (RFLPs), short tandem repeat (STR) analysis, single nucleotide polymorphism (SNP) analysis, DNA methylation analysis and so on. Nucleic acid extraction and purification is necessary for each of the DNA typing techniques, a downstream DNA typing may obtain effective typing data only when high-quality DNA is extracted from a forensic sample.

In DNA extraction, firstly, a cell has to be lysed to release chromatin in the cell nucleus; secondly, proteins in chromatin have to be digested and decomposed, so that the DNA is completely freed; and thirdly, impurities such as cell debris, inorganic salts and the like present in the solution have to be removed to purify the DNA. At present, in commercial DNA extraction kits, cell lysis and digestion is generally completed by using a surfactant and Proteinase K, and DNA purification is generally completed through reversible adsorption of DNA by a silica gel membrane, a magnetic bead and a silica bead under special solution conditions.

### Summary of the Invention

It is an object of the invention to provide a method for purifying and concentrating DNA in a forensic sample by using a selective filtration column, wherein residual human cells in the sample are lysed and digested by using a lysis solution, components such as cytomembranes, nuclear membranes, proteins and the like are digested, the DNA is then completely free in the solution, solid particles in the sample are centrifugally removed by a centrifuge tube, and the filtrate only comprises one bio-macromolecule, i.e., DNA, and some bio-micromolecules, the bio-macromolecule DNA is intercepted by the selective filtration column, while the micromolecular substances are filtered out, so that the micromolecule DNA in the filtrate is purified and concentrated, an ultrafiltration membrane is provided at a bottom of the selective filtration column, and under the condition of high-speed centrifugation, the ultrafiltration membrane is only permeable to micromolecular substances and not permeable to the bio-macromolecule DNA, so that the purification and concentration of the macromolecular substance DNA can be realized.

The object of the invention can be realized through the following technical solution:
a method for purifying and concentrating DNA in a forensic sample by using a selective filtration column, comprising the steps of:
step 1, adding cytosol after digestion and lysis into an inner tube of a selective filtration column, centrifuging the selective filtration column in a centrifuge of 3000-14000 r/min for 1-10 min, and filtering and removing micromolecular substances; and
step 2, adding 5-100 microliters of water into the selective filtration column, shaking for 30 seconds, transferring an aqueous solution in the selective filtration column into a clean centrifugal tube, in which case a solution in the centrifugal tube is purified and concentrated;
wherein the selective filtration column comprises an outer tube and an inner tube sleeved inside the outer tube, a liquid outlet is formed in a bottom of the inner tube, a liquid outlet pipe is integrally connected and fixed with the liquid outlet, a support plate is provided on an inner surface of a side wall of the inner tube above the liquid outlet, a plurality of filtrate holes are formed in a surface of the support plate, an ultrafiltration membrane and a sealing pressing ring are sequentially provided on the surface of the support plate up and down, and the sealing pressing ring is pressed against a surface of the ultrafiltration membrane.

Furthermore, the specific digestion and lysis to obtain the cytosol includes: (1) taking a forensic sample and adding into the lysis solution to carry out lysis and digestion for 15 min-12 h under 50-80°C; and (2) placing the cytosol after lysis and digestion into the centrifuge tube, centrifuging in the centrifuge of 3000-14000 r/min for 1-10 min, and then taking a supernatant in the centrifuge tube into the selective filtration column. Furthermore, the ultrafiltration membrane is a microfiltration membrane having a pore diameter ranging from 1 nm to 100 nm.

Furthermore, the ultrafiltration membrane is made of one of a group consisting of cellulose, cellulose derivatives, polyvinylidene fluoride, polysulfone, polyacrylonitrile, polyamide cross-linked polyvinyl alcohol, modified acrylic polymer, polycarbonate, polyvinyl chloride, polysulfonamide, and sulfonated polysulfone, or other materials that can be made into an ultrafiltration membrane having a pore diameter ranging from 1 nm to 20 nm.

The invention has the following advantageous effects:
According to the invention, residual human cells in the sample are lysed and digested by using a lysis solution, components such as cytomembranes, nuclear membranes, proteins and the like are digested, the DNA is then completely free in the solution, solid particles in the sample are centrifugally removed by a centrifuge tube, and the filtrate only comprises one bio-macromolecule, i.e., DNA, and some bio-micromolecules, the bio-macromolecule DNA is intercepted by the selective filtration column, while the micromolecular substances are filtered out, so that the micromolecule DNA in the filtrate is purified and concentrated, an ultrafiltration membrane is provided at a bottom of the selective filtration column, and under the condition of high-speed centrifugation, the ultrafiltration membrane is only permeable to micromolecular substances and not permeable to the bio-macromolecule DNA, so that the purification and concentration of the macromolecular substance DNA can be realized.

### Brief Description of the Drawings

In order that those skilled in the art may readily understand, the present invention will now be further described with reference to the accompanying drawings.
FIG. 1 is a typing profile of a DNA solution obtained by a method and a selective filtration column of the invention;
FIG. 2 is a cross-sectional view of the selective filtration column of the invention;
FIG. 3 is a schematic view showing a partial structure of the selective filtration column of the invention.

### Detailed Description of the Invention

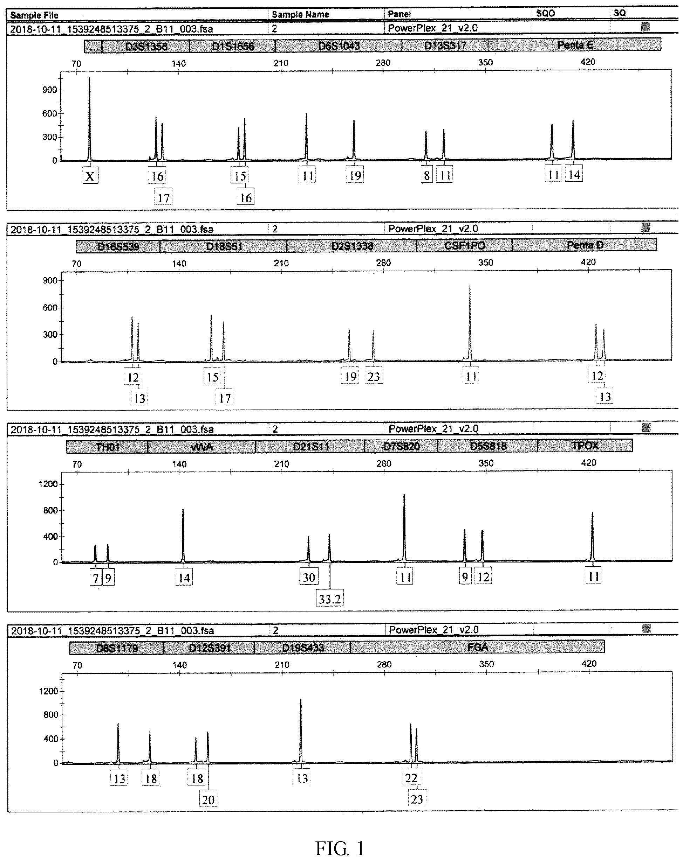

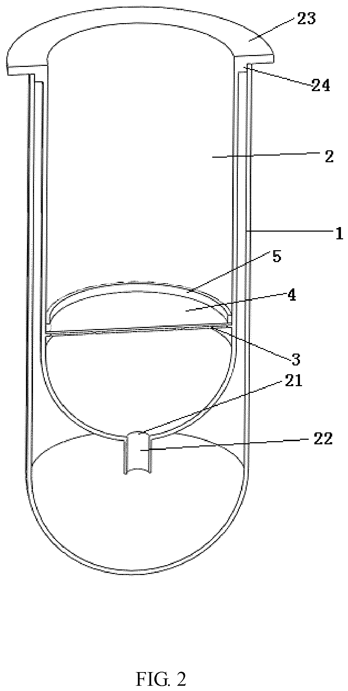

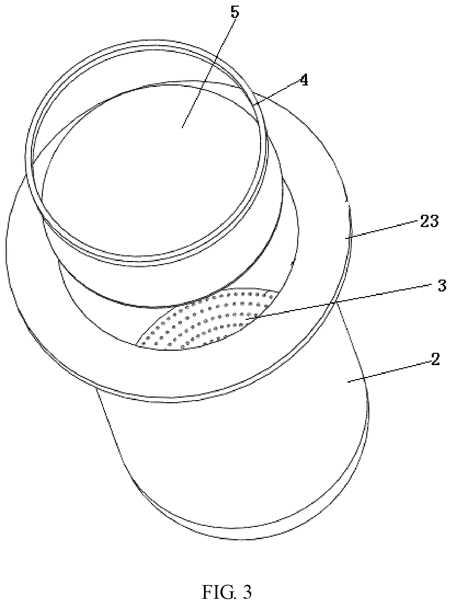

Referring to FIGS. 1 to 3, a detailed description is made in conjunction with the following embodiment:
The invention discloses a method for purifying and concentrating DNA in a forensic sample by using a selective filtration column, including the steps of:
step 1, taking a forensic sample and adding into a lysis solution to carry out lysis and digestion for 15 min-12 h under 50-80°C; and lysing and digesting human cells in the forensic sample by the lysis solution and releasing the DNA into the solution; wherein the lysis solution contains 1% (W/V) sodium dodecyl sulfate, 10 mmol/L disodium EDTA, 20 mmol/L tris (hydroxymethyl) aminomethane, 0.5 mg/ml Proteinase K, and the pH of the lysis solution is 7.5;
step 2, placing cytosol after lysis and digestion in step 1 into a centrifuge tube, centrifuging in a centrifuge of 3000-14000 r/min for 1-10 min, and then taking a supernatant in the centrifuge tube into an inner tube 2 of the selective filtration column;
step 3, centrifuging the selective filtration column in the centrifuge of 3000-14000 r/min for 1-10 min, and filtering and removing micromolecular substances; and
step 4, adding 5-100 microliters of water into the selective filtration column, shaking for 30 seconds, transferring an aqueous solution in the selective filtration column into a clean centrifuge tube, in which case a solution in the centrifuge tube is a purified and concentrated; performing PCR amplification on the obtained DNA solution by using a PowerPlex® 21 kit (produced by Promega), performing electrophoretic analysis on a PCR product by using an AB 3500 XL gene analyzer (produced by Applied Biosystems), analyzing the data by using Gene Mapper, and obtaining the results shown in FIG. 1;

The micromolecule impurities in the solution are removed by using an ultrafiltration membrane, so that the macromolecular DNA is purified and concentrated. The specific operation includes: taking a sample, lysing and digesting cells and proteins (the proteins are also macromolecules, the proteins are digested to become micromolecular amino acids or polypeptides), centrifuging to filter out the micromolecules by the ultrafiltration membrane, intercepting only the macromolecular DNA by the ultrafiltration membrane, adding water to shake and dissolve the DNA again, and obtaining a purified DNA solution.

The selective filtration column includes an outer tube 1 and an inner tube 2 sleeved inside the outer tube 1, a liquid outlet 21 is formed in a bottom of the inner tube 2, a liquid outlet pipe 22 is integrally connected and fixed with the liquid outlet 21, a support plate 3 is provided on an inner surface of a side wall of the inner tube 2 above the liquid outlet 21, and a plurality of filtrate holes are formed in a surface of the support plate 3; moreover, an ultrafiltration membrane 4 and a sealing pressing ring 5 are sequentially provided on the surface of the support plate 3 up and down, and the sealing pressing ring 5 is pressed against a surface of the ultrafiltration membrane 4 to prevent the ultrafiltration membrane 4 from sliding, therefore, the ultrafiltration membrane 4 is completely sealed and fixed, the solution is prevented from flowing out from a joint between the ultrafiltration membrane 4 and the inner tube 2 to cause pollution to lower layers; moreover, the inner tube 2 can be used for purifying and concentrating the solution containing no solid particles after lysis and digestion in a high-speed centrifugal state, wherein the purification and concentration means that micromolecules in the solution containing no solid particles pass through the ultrafiltration membrane in the high-speed centrifugal state, while the bio-macromolecule DNA is intercepted by the ultrafiltration membrane; the ultrafiltration membrane is a microfiltration membrane having pore diameter ranging from 1 nm to 100 nm, and the ultrafiltration membrane is made of one of a group consisting of cellulose, cellulose derivatives, polyvinylidene fluoride, polysulfone, polyacrylonitrile, polyamide cross-linked polyvinyl alcohol, modified acrylic polymer, polycarbonate, polyvinyl chloride, polysulfonamide, and sulfonated polysulfone, or other materials that can be made into an ultrafiltration membrane having a pore diameter ranging from 1 nm to 20 nm, macromolecules and micromolecules are separated by an ultrafiltration membrane having a pore diameter ranging from 2 nm to 100 nm under the pressure, in the ultrafiltration process, an aqueous solution flows over the surface of the ultrafiltration membrane under the pressure, solvents smaller than the pore diameter of the ultrafiltration membrane and micromolecular solutes pass through the ultrafiltration membrane under the pressure, and molecules larger than the pore diameter of the ultrafiltration membrane are intercepted;
a stopper ring 23 is integrally connected and fixed on an outer surface of a side wall of a top end of the inner tube 2, a fixing ring 24 is integrally connected and fixed on the outer surface of the side wall of the top end of the inner tube 2, the fixing ring 24 is tightly pressed and connected with an inner surface of a side wall of a top end of the outer tube 1, and a bottom surface of the stopper ring 23 is connected with a top end surface of the inner tube 2, so that the outer tube 1 is supported and suspended.

The preferred embodiments of the invention disclosed above are only used for illustrative purposes and do not describe all the details, nor do they limit the invention to the specific embodiments described. Apparently, according to the description, many modifications and changes can be made. This description selects and specifically describes these embodiments in order to better explain the principle and practical applications of the invention, so that those skilled in the art can readily understand and implement the invention. The invention is only defined by the claims in the full scope and equivalents thereof.

## Claims

1. A method for purifying and concentrating DNA in a forensic sample by using a selective filtration column, **characterized by** comprising the steps of:
step 1, adding cytosol after digestion and lysis into an inner tube of a selective filtration column, centrifuging the selective filtration column in a centrifuge of 3000-14000 r/min for 1-10 min, and filtering and removing micromolecular substances; and
step 2, adding 5-100 microliters of water into the selective filtration column, shaking for 30 seconds, transferring an aqueous solution in the selective filtration column into a clean centrifugal tube, in which case a solution in the centrifugal tube is purified and concentrated;
wherein the selective filtration column comprises an outer tube and an inner tube sleeved inside the outer tube, a liquid outlet is formed in a bottom of the inner tube, a liquid outlet pipe is integrally connected and fixed with the liquid outlet, a support plate is provided on an inner surface of a side wall of the inner tube above the liquid outlet, a plurality of filtrate holes are formed in a surface of the support plate, an ultrafiltration membrane and a sealing pressing ring are sequentially provided on the surface of the support plate up and down, and the sealing pressing ring is pressed against a surface of the ultrafiltration membrane.

2. The method for purifying and concentrating the DNA in the forensic sample by using the selective filtration column according to claim 1, **characterized in that** the specific digestion and lysis to obtain the cytosol comprises: (1) taking a forensic sample and adding into the lysis solution to carry out lysis and digestion for 15 min-12 h under 50-80°C; and (2) placing the cytosol after lysis and digestion into the centrifuge tube, centrifuging in the centrifuge of 3000-14000 r/min for 1-10 min, and then taking a supernatant in the centrifuge tube into the selective filtration column.

3. The method for purifying and concentrating the DNA in the forensic sample by using the selective filtration column according to claim 1, **characterized in that** the ultrafiltration membrane is a microfiltration membrane having a pore diameter ranging from 1 nm to 100 nm.

4. The method for purifying and concentrating the DNA in the forensic sample by using the selective filtration column according to claim 1, **characterized in that** the ultrafiltration membrane is made of one of a group consisting of cellulose, cellulose derivatives, polyvinylidene fluoride, polysulfone, polyacrylonitrile, polyamide cross-linked polyvinyl alcohol, modified acrylic polymer, polycarbonate, polyvinyl chloride, polysulfonamide, and sulfonated polysulfone, or other materials that can be made into an ultrafiltration membrane having a pore diameter ranging from 1 nm to 20 nm.
